Europäisches Patentamt

European Patent Office

Office européen des brevets

(19)

(11) Publication number: **0 155 189**
A2

# EUROPEAN PATENT APPLICATION

(12)

(21) Application number: 85301827.3

(22) Date of filing: 15.03.85

(51) Int. Cl.⁴: **C 12 N 15/00**, C 12 N 1/20,
C 12 N 1/06, C 12 N 9/36
// (C12N1/20, C12R1:19)

(30) Priority: 16.03.84 US 590138
11.09.84 US 649786

(43) Date of publication of application: 18.09.85
Bulletin 85/38

(84) Designated Contracting States: **AT BE CH DE FR GB IT
LI LU NL SE**

(71) Applicant: **GENENTECH, INC., 460 Point San Bruno
Boulevard, South San Francisco California 94080 (US)**

(72) Inventor: **Wetzel, Ronald Burnell, 455 Urbano Drive, San
Francisco California 94127 (US)**

(74) Representative: **Armitage, Ian Michael et al, MEWBURN
ELLIS & CO. 2/3 Cursitor Street, London EC4A 1BQ (GB)**

(54) Expression of cell wall degrading proteins and host cells harboring dna encoding such protein.

(57) A cell wall lytic enzyme is expressed in recombinant host cells without cell toxicity. The enzyme may be released at a predetermined point by a freeze and thaw cycle. This allows the expressed enzyme to act on the host cell walls and lyse the cells, thereby facilitating recovery of the enzyme or other intracellular products.

EP 0 155 189 A2

1

EXPRESSION OF CELL WALL DEGRADING PROTEINS

AND HOST CELLS HARBORING DNA ENCODING SUCH

PROTEIN

The present invention relates to the high level non-toxic expression of cell wall disrupting protein and means and methods for said expression. The invention further relates to the methods and means by which to lyse cell walls at will thereby providing a mechanism facilitating recovery of intracellular proteins.

Lysozyme is one of several compounds responsible for disrupting or lysing cell walls. Thus, lysozyme is toxic in nature. For example, it is known that bacterial viruses such as T4 phage kill bacteria using lysozyme to destroy the bacterial cell wall. Because of its toxic properities, it has heretofore been unknown to achieve high-level lysozyme expression in host cell culture, and further unknown to extract lysozyme in useful quantities from host cell cultures.

The present invention provides a means for inducing a recombinant cell culture to produce lysozymes, which would ordinarily be expected to kill such host cells by means of destructing or lysing the cell wall structure. The production of lysozymes in accordance herewith is achieved with the unexpected result that the viability of the cells is not inhibited or destroyed so that useful quantities of the product, lysozyme, can be obtained.

Thus, the present invention relates to the preparation of the polypeptide lysozyme in a recombinant host cell in which it would ordinarily function as a lyse/kill agent of the cells. The product lysozyme, as a product of recombinant DNA technology, as well as associated cloning vehicles and replicable expression vectors and the host cell harboring such vectors are covered by the present invention, in all of its aspects. One aspect of the invention that is especially useful is the high level non-toxic expression of products ordinarily toxic to the cell. Toxicity can be induced at any time during the non-toxic expression of such products by means of a freeze/thaw cycle. Thus, this invention provides a means by which to lyse cell walls at will thereby providing a mechanism facilitating recovery of intracellular products, including other recombinant products such as human growth hormone; various interferons, e.g., IFN-gamma, tissue plasminogen activator, hepatitis B surface antigen, and so on.

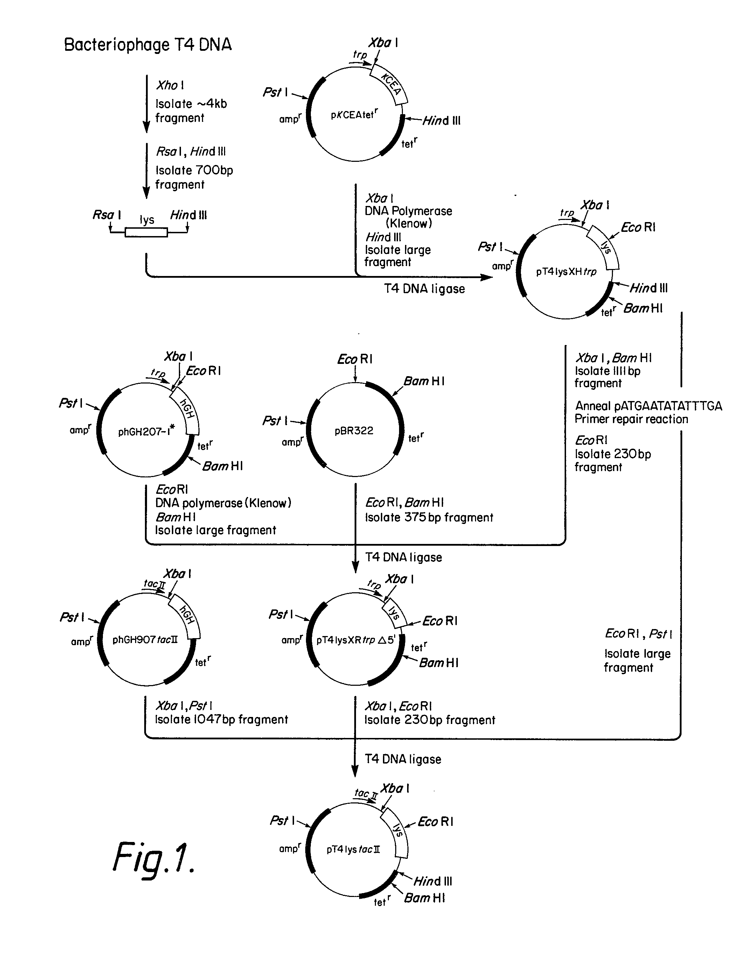

Figure 1 illustrates the method for constructing the plasmid pT4lystacII.

3

## Construction of pT4lysXHtrp

The accompanying drawing illustrates the construction of the plasmid pT4lystacII. A sample of cytosine containing T4 DNA (1, 2) was digested with the restriction endonuclease XhoI and a fragment of about 4,000 base pairs was isolated by agarose gel electrophoresis. The purified fragment was then cleaved with the restriction endonucleases RsaI and HindIII to yield a gel-purified fragment of about 700 base pairs which contained the T4 lysozyme gene. The plasmid pKCEAtetRXAP (3) was cleaved with the restriction endonuclease XbaI, filled in with DNA polymerase I (Klenow fragment) and subsequently digested with the restriction endonuclease HindIII. The gel purified vector fragment was ligated with T4 DNA ligase to the 700 bp fragment to yield the plasmid pT4lysXHtrp.

## Construction of pT4lysXRtrpΔ5'

The plasmid phGH207-1* (4) was cleaved with the restriction endonuclease EcoRI and elongated with DNA polymerase I (Klenow fragment), then cleaved with the restriction endonuclease BamHI, and the large vector fragment was purified by gel electrophoresis. The plasmid pT4lysXHtrp was cleaved with the restriction endonucleases XbaI and BamHI to yield a 1111 base pair fragment. A 14-base oligonucleotide primer was used in a primer repair reaction to delete the 97 bases of untranslated T4 DNA on the 5' end of the lysozyme gene (5). About 0.5 micrograms of the primer was labeled using $^{32}$P-ATP and polynucleotide kinase. The gel purified XbaI-BamHI fragment of pT4lysXHtrp was annealed to the labeled primer by heating to

4

95-100°C for 4 minutes followed by quick freezing. Nucleotide triphosphates were then added to the frozen mixture, which was then allowed to thaw. When the mix was partially thawed, DNA polymerase I (Klenow fragment) was added. The reaction was incubated at 37°C for 1.5 hours. EDTA was added to stop the reaction. The DNA was washed with phenol and chloroform, precipitated and washed with ethanol. The fragment was identified on an acrylamide gel by autoradiography. The identified fragment was eluted from the gel and quantified by scintillation counting. The resulting fragment was then digested with the restriction endonuclease EcoRI to yield a fragment of 230 base pairs which was isolated by gel electrophoresis. The plasmid pBR322 was digested with the restriction endonucleases EcoRI and BamHI and the 375 base pair fragment purified by gel electrophoresis. A three-way ligation reaction was performed with the three fragments to construct the pT4lysXRtrp$\Delta$5' plasmid.

This ligation mixture was used to transform E. coli K-12 294 (ATCC No. 31446). Plasmid DNA was isolated from the resulting transformants and applied to nitrocellulose paper. The paper was treated with $^{32}$P labeled primer. When the nitrocellulose was gradually washed under conditions of increasing hybridization stringency, 37 positive sequences were seen. Six of the most strongly hybridizing positives were picked for M13 sequencing.

The plasmid isolated from each of these strongly hybridizing clones was digested with the restriction endonucleases EcoRI and XbaI and the 230 base pair fragment was gel purified. The double stranded RF form of the M13mp10 DNA

0155189

5

was also digested with EcoRI and XbaI. The 230 base pair fragment was ligated into the digested M13 vector fragment overnight at room temperature. The ligation reaction mixtures were used to transfect E. coli JM101 (ATCC No. 33876), and plaques were picked and sequenced by standard methods. Only one of the positives analyzed by DNA sequencing had the correct sequence. The replicable expression vehicle which contains the EcoRI/XbaI fragment of the correct sequence was designated pT4lysXRtrpΔ5'.

Construction of pT4lystacII

A 230 base pair fragment of the replicable cloning vehicle pT4lysXRtrpΔ5' containing the 5' half of the lysozyme gene was isolated after treatment of the replicable cloning vehicle with the restriction with the restriction endonucleases XbaI/EcoRI and was cloned into the phage M13mp10. This fragment was ligated to the 1047 base pair fragment cleaved from the replicable cloning vehicle phGH907tacII with the restriction endonucleases XbaI and PstI, and to the large fragment of the replicable expression vector pT4LysXHtrp which had been cleaved with EcoRI and PstI, generating the replicable expression vector pT4lystacII. This replicable expression vector used to transform E. coli D1210 (ATCC No. 31449). The transformants were grown and selected on LB/ampicillin plates. Replicable expression vectors (REVs) were isolated from cells grown from the colonies and analyzed by restriction mapping. Colonies containing REVs with restriction fragments of the expected size, consistent with the desired final construction, were again grown. A SDS polyacrylamide gel with crude cellular

6

protein from each IPTG induced culture indicated the presence or absence of an induced protein of about 19,000 molecular weight. When it was later established that the T4 lysozyme isolated from a culture of these cells contained active lysozyme, the REV isolated from these cells was designated pT4lystacII.

A colony of E. coli D1210/pT4lystacII was picked from a culture plate (LB broth/ampicillin) from a fresh transformation and grown overnight at 37° in five ml of LB (6) broth containing 20 micrograms/1 ampicillin. This culture was used to inoculate 1 liter of M9 medium (6) containing 20 micrograms/ml ampicillin in a 4 liter shake flask. When the culture reached an $OD_{550}$ of about 2, or late log phase growth, it was induced with the addition of 1mM isopropyl-beta-D-thiogalactoside (6). After one hour the cells were harvested by centrifugation and frozen.

Frozen cells from 0.5 L of culture were lysed by addition to 20 ml 50 mM tris HCl, pH 7.5, 1 mM EDTA, 1 mM 2-mercaptoethanol, 1 mM phenylmethylsulfonyl fluoride on ice. Two mls of five percent polyethylene imine hydrochloride (pH 7.5) was added with stirring and the suspension centrifuged at 10,000 rpm for 20 minutes in a Sorvall SS-34 rotor. The supernatant was passed over a DEAE-cellulose column and washed with 50 mM tris HCl, pH 7.5, 1 mM EDTA, 3 mM 2-mercaptoethanol.

The flowthrough fractions containing 0.3 $OD_{280}$ or higher were pooled and adjusted to pH 5.2 with concentrated acetic acid. This was loaded onto a CM-cellulose column (10 ml bed volume in a 1 cm diameter column at 4°) equilibrated with 50 mM NaOAc, pH 5.5, 1 mM EDTA, 3 mM 2-mercaptoethanol. The

7

column was washed with equilibration buffer, then eluted with a linear gradient of 0-0.5 M NaCl in equilibration buffer, total volume 100 ml, at a flow rate of 10 ml/hr. The fractions containing lysozyme activity (6) eluted at a NaCl concentration of about 0.25 M. These fractions contained as the major species a protein shown by SDS polyacrylamide gel electrophoresis to be of molecular weight about 19,000. The yield of protein was 4 milligrams.

The molecular weight found for the recombinant T4 lysozyme was as expected based on the sequence of T4 lysozyme and its enzymatic activity as measured in the turbidity assay and compared to the activity of egg white lysozome. This was supported by amino acid compositional analysis.

8

## BIBLIOGRAPHY

The following references are cited in the foregoing specification by parenthetical numerals corresponding to those listed below. All of these references are hereby incorporated by reference.

1.  O' Farrell, et al., Molec. Gen. Genet. 179, 421-435.

2.  Owen, et al., J. Mol. Biol. 165, 229-248.

3.  Cabilly, Shmuel, et al., Generation of Antibody Activity from Immunoglobulin Polypeptide Chains Produced in Escherichia coli Proc. Natl. Acad. Sci. (USA), 81, 3273.

4.  Gray, et al., Bio/Technology, 2, 161- 165.

5.  Goeddel, et al., Nucleic Acids Res., 8 4057-4074.

6.  Miller, J. H., Experiments in Molecular Genetics, (1972), Cold Spring Harbor Laboratory.

0155189

CLAIMS

1. A transformed host cell harboring a replicable expression vector comprising DNA encoding a cell wall disrupting protein which ordinarily kills said host cell.

2. The host cell of claim 1 wherein the cell wall disrupting protein is lysozyme.

3. The host cell of claim 2 wherein the lysozyme is T4 lysozyme.

4. The host cell of any one of claims 1, 2 and 3 which is E. coli.

5. The host cell of claim 4 which is E. coli strain D1210.

6. A replicable expression vector harboring the DNA of any one of claims 1 to 5.

7. A method of directing the non-toxic expression of a cell wall disrupting protein in a host cell ordinarily killed by said protein comprising:

(a) forming a replicable expression vector of claim 6;

(b) transforming said host cell with the replicable expression vector; and

(c) culturing the transformed host cell to produce said cell wall disrupting protein.

8.   As a product of recombinant DNA technology, lysozyme.

9.   A process for recovering a recombinant polypeptide from a host cell within which it is produced comprising coproducing a cell wall disrupting protein therein followed by subjecting the host cell to freeze/thaw cycle.

CLAIMS

1.    A method of directing the non-toxic expression of a cell wall disrupting protein in a host cell ordinarily killed by said protein comprising:

(a) forming a replicable expression vector comprising DNA encoding said cell wall disrupting protein;

(b) transforming said host cell with the replicable expression vehicle; and

(c) culturing the transformed host cell to product said cell wall disrupting protein.


2.    The method of claim 1 wherein the cell wall disrupting protein is lysozyme.


3.    The method of claim 2 wherein the lysozyme is T4 lysozyme.


4.    The method of any one of claims 1, 2 and 3 wherein the host cell is E. coli.


5.    The method of claim 4 wherein the E. coli is strain D1210.


6.    A process for recovering a recombinant polypeptide from a host cell within which it is produced comprising coproducing a cell wall disrupting protein therein followed by

10

0155189

subjecting the host cell to freeze/thaw cycle.

Fig.1.